# EUROPEAN PATENT APPLICATION

(11) **EP 1 961 749 A1**
(43) Date of publication of application: **27.08.2008**
(21) Application number: 06833454.9
(22) Date of filing: 28.11.2006
(51) Int. Cl.: C07D 401/12, A61K 31/4427, A61P 9/00, A61P 9/04, A61P 9/10, A61P 9/12, A61P 13/12

(54) **ACID ADDITION SALT OF DIHYDROPYRIDINE DERIVATIVE**

(30) Priority: 29.11.2005 JP 2005344257
(71) Applicant: Daiichi Sankyo Company, Limited, Chuo-ku Tokyo 103-8426 (JP); Ube Industries, Ltd., Ube-Shi, Yamaguchi 755-8633 (JP)
(72) Inventor: HAGIHARA, Masahiko, Yamaguchi 755-8633 (JP); SHIMIZU, Motohisa, Yamaguchi 755-8633 (JP); SETA, Yasuo, Tokyo 140-8710 (JP); KOBAYASHI, Katsuhiro, Tokyo 140-8710 (JP); YOSHIGAE, Yasushi, Tokyo 140-8710 (JP)
(74) Representative: HOFFMANN EITLE
(86) International application number: PCT/JP2006/323650
(87) International publication number: WO 2007/063822

(57) **Abstract**

There is provided an excellent medicine for treating or preventing hypertension or the like. A specific acid addition salt of 2-amino-1,4-dihydro-6-methyl-4-(3-nitrophenyl)-3,5-pyridinedicarboxylic acid 3-(1-diphenylmethylazetidin-3-yl) ester 5-isopropyl ester is useful as a medicine for treating or preventing hypertension or the like.

## Description

### TECHNICAL FIELD

The present invention relates to a specific acid addition salt of 2-amino-1,4-dihydro-6-methyl-4-(3-nitrophenyl)-3,5-pyridinedicarboxylic acid 3-(1-diphenylmethyl-azetidin-3-yl) ester 5-isopropyl ester having excellent calcium antagonistic effect, hypotensive effect, vasodilative effect, cardioprotective effect, antiarteriosclerotic effect, diuretic effect, nephropathy inhibitory effect and lipid peroxide generation inhibitory effect;
a pharmaceutical composition containing a specific acid addition salt of 2-amino-1,4-dihydro-6-methyl-4-(3-nitrophenyl)-3,5-pyridinedicarboxylic acid 3-(1-diphenyl-methylazetidin-3-yl) ester 5-isopropyl ester as an active ingredient, preferably a pharmaceutical composition for treating or preventing hypertension, heart disease, arteriosclerosis or nephropathy, more preferably a pharmaceutical composition for treating or preventing hypertension or heart disease, and most preferably a pharmaceutical composition for treating or preventing hypertension;
a method for treating or preventing a disease, preferably hypertension, heart disease, arteriosclerosis or nephropathy, more preferably hypertension or heart disease, and most preferably hypertension, the method comprising administering a pharmacologically effective amount of a specific acid addition salt of 2-amino-1,4-dihydro-6-methyl-4-(3-nitrophenyl)-3,5-pyridinedicarboxylic acid 3-(1-diphenylmethylazetidin-3-yl) ester 5-isopropyl ester to a warm-blooded animal (preferably a human); and
a method for producing a specific acid addition salt of 2-amino-1,4-dihydro-6-methyl-4-(3-nitrophenyl)-3,5-pyridinedicarboxylic acid 3-(1-diphenylmethylazetidin-3-yl) ester 5-isopropyl ester.

BACKGROUND ART

2-Amino-1,4-dihydro-6-methyl-4-(3-nitrophenyl)-3,5-pyridinedicarboxylic acid 3-(1-diphenylmethylazetidin-3-yl) ester 5-isopropyl ester [hereinafter may be referred to as "compound (I)"] that is a dihydropyridine calcium antagonist is known to have pharmacological activities such as a calcium antagonistic effect and hypotensive effect and to be useful as a medicine for treatment of hypertension or the like (see Patent Document 1 or 2). Further, compounds having a calcium antagonistic effect are known to be useful as therapeutic agents for heart disease, arteriosclerosis or nephropathy [for example, (i) Goodman & Gilman's The pharmacological basis of therapeutics, chapter 32, p.767-774; (ii) Annual Report of Sankyo Research Laboratories, 2002, vol.54, p.1-64; (iii) The American Journal of Medicine, 1989, vol.86 (suppl 4A), p.27-32; and (iv) The American Journal of Hypertention, 1993, vol.6, p.251S-259S].

A dihydrochloride of the compound (I) is known as an acid addition salt of the compound (I) (see Patent Document 1 or 2), but other acid addition salts are not known. The dihydrochloride is produced by a method using hydrochloric acid gas and is an amorphous solid. An acid addition salt of the compound (I) which can be obtained as a crystalline solid has not yet been known.

It is useful to find a compound having properties such as solubility, oral absorbability, concentration in blood and bioavailability (BA) superior to those of the free compound (I). It is also useful to find an acid addition salt of the compound (I) which can be obtained as a crystalline solid in order to supply a pharmaceutical compound having a certain quality in an industrial scale.
Patent Document 1: JP 3-31715A
Patent Document 2: U.S. Patent No. 4,772,596

### DISCLOSURE OF THE INVENTION

### PROBLEMS TO BE SOLVED BY THE INVENTION

The present inventors have conducted extensive studies on acid addition salts of the compound (I) and have found that a specific acid addition salt of the compound (I) has excellent calcium antagonistic effect and hypotensive effect, for example, and is excellent as a pharmaceutical compound in terms of properties such as bioavailability, crystallinity and thermal stability, and is therefore useful as a medicine, in particular, a medicine for treating or preventing hypertension or the like. This finding has led to the completion of the present invention.

MEANS TO SOLVE THE PROBLEMS

The present invention provides a specific acid addition salt of 2-amino-1,4-dihydro-6-methyl-4-(3-nitrophenyl)-3,5-pyridinedicarboxylic acid 3-(1-diphenylmethylazetidin-3-yl) ester 5-isopropyl ester having excellent calcium antagonistic effect, hypotensive effect, vasodilative effect, cardioprotective effect, antiarteriosclerotic effect, diuretic effect, nephropathy inhibitory effect and lipid peroxide generation inhibitory effect;
a pharmaceutical composition containing a specific acid addition salt of 2-amino-1,4-dihydro-6-methyl-4-(3-nitrophenyl)-3,5-pyridinedicarboxylic acid 3-(1-diphenylmethylazetidin-3-yl) ester 5-isopropyl ester as an active ingredient, preferably a pharmaceutical composition for treating or preventing hypertension, heart disease, arteriosclerosis or nephropathy, more preferably a pharmaceutical composition for treating or preventing hypertension or heart disease, and most preferably a pharmaceutical composition for treating or preventing hypertension;
a method for treating or preventing a disease, preferably hypertension, heart disease, arteriosclerosis or nephropathy, more preferably hypertension or heart disease, and most preferably hypertension, the method comprising administering a pharmacologically effective amount of a specific acid addition salt of 2-amino-1,4-dihydro-6-methyl-4-(3-nitrophenyl)-3,5-pyridinedicarboxylic acid 3-(1-diphenylmethylazetidin-3-yl) ester 5-isopropyl ester to a warm-blooded animal (preferably a human); and
a method for producing a specific acid addition salt of 2-amino-1,4-dihydro-6-methyl-4-(3-nitrophenyl)-3,5-pyridinedicarboxylic acid 3-(1-diphenylmethylazetidin-3-yl) ester 5-isopropyl ester.

In one aspect, the present invention is based on the following finding.

(i) A specific acid addition salt of the compound (I) has pharmacokinetic properties (in particular, bioavailability and concentration in blood) superior to those of the free compound (I) or another acid addition salt of the compound (I).

In another aspect, the present invention is based on the following findings.

(ii) The compound (I) forms an acid addition salt with a specific acid but does not form an acid addition salt with another acid. That is, the compound (I) preferentially forms a salt with a specific acid.

(iii) A specific acid addition salt of the compound (I) can be obtained as a crystalline solid, but another acid addition salt can be obtained only as an amorphous solid. That is, a specific acid addition salt of the compound (I) has crystallinity superior to that of another acid addition salt.

(iv) Acid addition salts of the compound (I) which can be obtained differ from each other in terms of thermal stability. That is, a specific acid addition salt of the compound (I) has thermal stability superior to that of another acid addition salt.

In still another aspect, the present invention is based on the following finding.

(v) Since the compound (I) has two ester groups, it is usually expected that the ester residue elimination by hydrolysis occurs in the presence of a strong acid and water. Further, since an acid addition salt is generally easily dissolved in water, it is usually expected to be difficult to obtain an acid addition salt in the presence of water. However, despite these expectations, a specific acid addition salt of the compound (I) can be obtained as a crystalline solid with good yield under the reaction conditions where a strong acid and water are present.

It is difficult to anticipate any of the aforementioned findings (i) to (v) from the prior art which has already been known.

In one aspect, the present invention provides:
(1) A hydrobromide, citrate, methanesulfonate, benzenesulfonate, p-toluenesulfonate or naphthalenesulfonate of 2-amino-1,4-dihydro-6-methyl-4-(3-nitrophenyl)-3,5-pyridinedicarboxylic acid 3-(1-diphenylmethylazetidin-3-yl) ester 5-isopropyl ester;
(2) The salt compound according to (1), which is a hydrobromide, methanesulfonate or p-toluenesulfonate of 2-amino-1,4-dihydro-6-methyl-4-(3-nitrophenyl)-3,5-pyridinedicarboxylic acid 3-(1-diphenylmethylazetidin-3-yl) ester 5-isopropyl ester;
(3) The salt compound according to (2), which is a hydrobromide of 2-amino-1,4-dihydro-6-methyl-4-(3-nitrophenyl)-3,5-pyridinedicarboxylic acid 3-(1-diphenylmethylazetidin-3-yl) ester 5-isopropyl ester;
(4) The salt compound according to (3), which is a dihydrobromide of 2-amino-1,4-dihydro-6-methyl-4-(3-nitrophenyl)-3,5-pyridinedicarboxylic acid 3-(1-diphenylmethylazetidin-3-yl) ester 5-isopropyl ester;
(5) A crystal of the dihydrobromide of 2-amino-1,4-dihydro-6-methyl-4-(3-nitrophenyl)-3,5-pyridinedicarboxylic acid 3-(1-diphenylmethylazetidin-3-yl) ester 5-isopropyl ester according to (4);
(6) The crystal according to (5), which shows main d spacing peaks at 16, 4.4, 3.9, 3.1 and 3.0 Å in a powder X-ray diffraction pattern obtained by irradiation with Cu Kα rays;
(7) The salt compound according to (2), which is a methanesulfonate of 2-amino-1,4-dihydro-6-methyl-4-(3-nitrophenyl)-3,5-pyridinedicarboxylic acid 3-(1-diphenylmethylazetidin-3-yl) ester 5-isopropyl ester;
(8) The salt compound according to (7), which is a dimethanesulfonate of 2-amino-1,4-dihydro-6-methyl-4-(3-nitrophenyl)-3,5-pyridinedicarboxylic acid 3-(1-diphenylmethylazetidin-3-yl) ester 5-isopropyl ester;
(9) A crystal of the dimethanesulfonate of 2-amino-1,4-dihydro-6-methyl-4-(3-nitrophenyl)-3,5-pyridinedicarboxylic acid 3-(1-diphenylmethylazetidin-3-yl) ester 5-isopropyl ester according to (8);
(10) The crystal according to (9), which shows main d spacing peaks at 12, 7.8, 6.1, 4.8 and 4.5 Å in a powder X-ray diffraction pattern obtained by irradiation with Cu Kα rays;
(11) The salt compound according to (7), which is a trimethanesulfonate of 2-amino-1,4-dihydro-6-methyl-4-(3-nitrophenyl)-3,5-pyridinedicarboxylic acid 3-(1-diphenylmethylazetidin-3-yl) ester 5-isopropyl ester;
(12) A crystal of the trimethanesulfonate of 2-amino-1,4-dihydro-6-methyl-4-(3-nitrophenyl)-3,5-pyridinedicarboxylic acid 3-(1-diphenylmethylazetidin-3-yl) ester 5-isopropyl ester according to (11);
(13) The crystal according to (12), which shows main d spacing peaks at 11, 4.6, 4.4, 3.9 and 3.6 Å in a powder X-ray diffraction pattern obtained by irradiation with Cu Kα rays;
(14) The salt compound according to (2), which is a p-toluenesulfonate of 2-amino-1,4-dihydro-6-methyl-4-(3-nitrophenyl)-3,5-pyridinedicarboxylic acid 3-(1-diphenylmethylazetidin-3-yl) ester 5-isopropyl ester;
(15) The salt compound according to (14), which is a di-p-toluenesulfonate of 2-amino-1,4-dihydro-6-methyl-4-(3-nitrophenyl)-3,5-pyridinedicarboxylic acid 3-(1-diphenylmethylazetidin-3-yl) ester 5-isopropyl ester;
(16) A crystal of the di-p-toluenesulfonate of 2-amino-1,4-dihydro-6-methyl-4-(3-nitrophenyl)-3,5-pyridinedicarboxylic acid 3-(1-diphenylmethylazetidin-3-yl) ester 5-isopropyl ester according to (15); or
(17) The crystal according to (16), which shows main d spacing peaks at 6.8, 4.9, 4.7 and 4.2 Å in a powder X-ray diffraction pattern obtained by irradiation with Cu Kα rays.

In the present invention, 2-amino-1,4-dihydro-6-methyl-4-(3-nitrophenyl)-3,5-pyridinedicarboxylic acid 3-(1-diphenylmethylazetidin-3-yl) ester 5-isopropyl ester is a compound having the following structural formula (Ia).

[Formula 1]

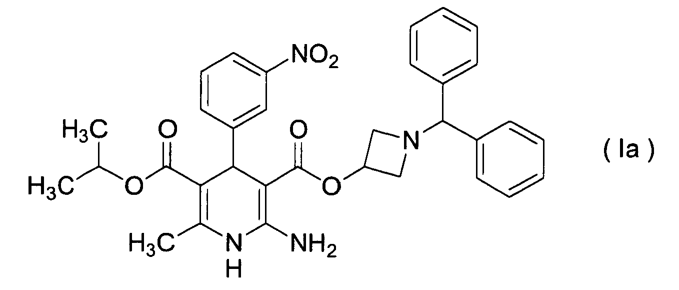

In the present invention, the acid moiety in the acid addition salt of the compound (I) is not particularly limited insofar as the acid moiety is an acid that can form an acid addition salt with the compound (I). The acid moiety may be mentioned, for example, hydrochloric acid, hydrobromic acid, hydriodic acid, sulfuric acid, nitric acid, phosphoric acid, acetic acid, oxalic acid, malonic acid, fumaric acid, maleic acid, tartaric acid, succinic acid, citric acid, methanesulfonic acid, benzenesulfonic acid, p-toluenesulfonic acid or naphthalenesulfonic acid, preferably hydrochloric acid, hydrobromic acid, sulfuric acid, phosphoric acid, fumaric acid, tartaric acid, citric acid, methanesulfonic acid, benzenesulfonic acid, p-toluenesulfonic acid or naphthalenesulfonic acid, more preferably hydrobromic acid, citric acid, methanesulfonic acid, benzenesulfonic acid, p-toluenesulfonic acid or naphthalenesulfonic acid, still more preferably hydrobromic acid, methanesulfonic acid or p-toluenesulfonic acid, yet more preferably hydrobromic acid or methanesulfonic acid, and most preferably hydrobromic acid.

In the present invention, the compound (I) has three basic groups (amino, azetidin-3-yl and dihydropyridyl groups). In the acid addition salt formed by the compound (I) and a monovalent, divalent or trivalent acid, the molar ratio of the compound (I) to the acid [compound (I)/acid] may be 1/1, 1/2 or 1/3 in the case of the monovalent acid, 2/1, 1/1 or 2/3 in the case of the divalent acid, for example, and 3/1, 3/2 or 1/1 in the case of the trivalent acid, for example, respectively. Such individual acid addition salts and mixtures thereof are included in the present invention.

In the present invention, the hydrobromide of the compound (I) includes a monohydrobromide, dihydrobromide and trihydrobromide and is preferably a dihydrobromide. The citrate of the compound (I) includes a 1/3 citrate, 2/3 citrate and monocitrate, for example, and is preferably a monocitrate. The methanesulfonate of the compound (I) includes a monomethanesulfonate, dimethanesulfonate and trimethanesulfonate and is preferably a dimethanesulfonate or trimethanesulfonate, and most preferably a trimethanesulfonate. The benzenesulfonate of the compound (I) includes a monobenzenesulfonate, dibenzenesulfonate and tribenzenesulfonate and is preferably a dibenzenesulfonate. The p-toluenesulfonate of the compound (I) includes a mono-p-toluenesulfonate, di-p-toluenesulfonate and tri-p-toluenesulfonate and is preferably a di-p-toluenesulfonate. The naphthalenesulfonate of the compound (I) includes a mononaphthalenesulfonate, dinaphthalenesulfonate and trinaphthalenesulfonate and is preferably a dinaphthalenesulfonate.

In the present invention, the triacid salt includes:
(i) a salt formed by the compound (I) having three basic groups protonated (1 mol) and an acid from which one proton dissociates (3 mol);
(ii) an adduct formed by a salt formed by the compound (I) having two basic groups protonated (1 mol) and an acid from which one proton dissociates (2 mol) and an acid from which a proton does not dissociate (1 mol); and
(iii) an adduct formed by a salt formed by the compound (I) having one basic group protonated (1 mol) and an acid from which one proton dissociates (1 mol) and an acid from which a proton does not dissociate (2 mol).
The triacid salt is preferably a salt shown in (i) above or an adduct shown in (ii) above, and most preferably an adduct shown in (ii) above. For example, the trimethanesulfonate of the compound (I) includes:
(i-1) a salt formed by the compound (I) having three basic groups protonated (1 mol) and a methanesulfonate anion (MeSO₃⁻) (3 mol);
(ii-1) an adduct formed by a salt formed by the compound (I) having two basic groups protonated (1 mol) and a methanesulfonate anion (2 mol) and methanesulfonic acid (1 mol); and
(iii-1) an adduct formed by a salt formed by the compound (I) having one basic group protonated (1 mol) and a methanesulfonate anion (1 mol) and methanesulfonic acid (2 mol).
The trimethanesulfonate is preferably a salt shown in (i-1) above or an adduct shown in (ii-1) above, and most preferably an adduct shown in (ii-1) above.

In the present invention, the acid addition salt of the compound (I) may be present as a hydrate or solvate. Such individual hydrates or solvates or mixtures thereof are included in the present invention. In the present invention, the hydrate includes a hydrate containing any amount of water (for example, a hemihydrate, monohydrate or dihydrate), and the solvate includes a solvate containing any amount of a solvent (for example, a hemisolvate, monosolvate or disolvate).

In the present invention, the acid addition salt of the compound (I) has one asymmetric center and may exist as an optical isomer. Such individual isomers and mixtures thereof are each described by a single formula such as the formula (Ia). The present invention includes any of these individual isomers and mixtures thereof at any ratio (including racemates).

In the present invention, the acid addition salt of the compound (I) or a hydrate or solvate thereof may form a crystal having a plurality of different internal structures and physicochemical properties (crystal polymorphism) depending on the reaction conditions and crystallization conditions. Such individual crystals or mixtures thereof at any ratio are included in the present invention. A crystalline solid and an amorphous solid may be mixed. Such a mixture at any ratio is included in the present invention. That is, the crystal of the present invention having a specific crystal form may contain a crystal having another crystal form or an amorphous solid. The content of the specific crystal form is preferably 50% or more, more preferably 80% or more, still more preferably 90% or more, yet more preferably 93% or more, particularly preferably 95% or more, and most preferably 97% or more.

In the present invention, the crystal represents a solid having an internal structure three-dimensionally formed by regular repetition of constituent atoms (or groups of constituent atoms) and is distinguished from an amorphous solid not having such a regular internal structure. Whether or not a solid is a crystal can be examined by a crystallographically known method (such as powder X-ray crystallography and differential scanning calorimetry). For example, in powder X-ray crystallography of a solid using X-rays obtained by irradiation with Cu Kα rays, the solid is determined to be a crystal when a specific peak is observed in its X-ray diffraction pattern, and the solid is determined to be amorphous when a specific peak is not observed. The solid is determined to be a crystal having a low degree of crystallinity when the peak can be read but is not clear (for example, broad); such a crystal having a low degree of crystallinity is also included in the crystal of the present invention.

In powder X-ray crystallography using Cu Kα rays, a sample is usually irradiated with Cu Kα rays (in which Kα1 and Kα2 rays are not separated). An X-ray diffraction pattern can be obtained by analyzing diffraction derived from Kα rays, or alternatively can be obtained by analyzing only diffraction derived from Kα1 rays taken from diffraction derived from Kα rays. In the present invention, the powder X-ray diffraction pattern obtained by irradiation with Kα rays includes an X-ray diffraction pattern obtained by analyzing diffraction derived from Kα rays and an X-ray diffraction pattern obtained by analyzing diffraction derived from Kα1 rays and is preferably an X-ray diffraction pattern obtained by analyzing diffraction derived from Kα1 rays.

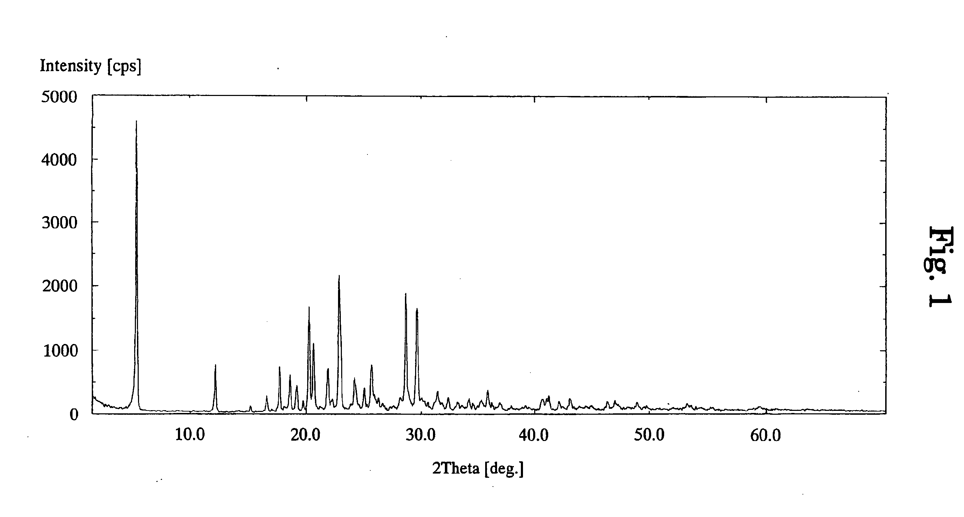

In the following powder X-ray diffraction patterns of Figures 1 to 11, the vertical axis indicates a diffraction intensity [counts/seconds (cps)] and the horizontal axis indicates a diffraction angle 2θ (°). The d spacing (Å) can be calculated by the formula 2dsinθ = nλ where n = 1. In the above formula, Kα rays have a wavelength λ of 1.54 Å and Kα1 rays have a wavelength λ of 1.541 Å. Since the position and relative intensity of the d spacing may change depending on the measurement conditions and the like, identity of the crystal form should be recognized with reference to the entire spectral pattern appropriately even when the d spacing slightly differs.

The crystal of the dihydrobromide of the compound (I) of the present invention may be a crystal showing main d spacing peaks at 16, 4.4, 3.9, 3.1 and 3.0 Å in a powder X-ray diffraction pattern obtained by irradiation with Cu Kα rays which is shown in Figure 1, for example. Here, the main peak is a peak having a relative intensity of 30 or more when a peak of d spacing at 16 Å has an intensity of 100.

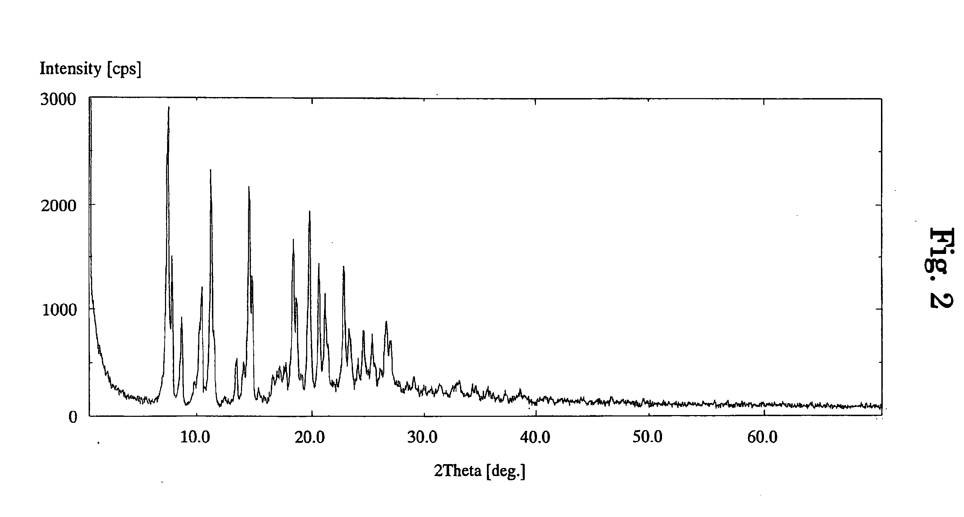

The crystal of the dimethanesulfonate of the compound (I) of the present invention may be a crystal showing main d spacing peaks at 12, 7.8, 6.1, 4.8 and 4.5 Å in a powder X-ray diffraction pattern obtained by irradiation with Cu Kα rays which is shown in Figure 2, for example. Here, the main peak is a peak having a relative intensity of 55 or more when a peak of d spacing at 12 Å has an intensity of 100.

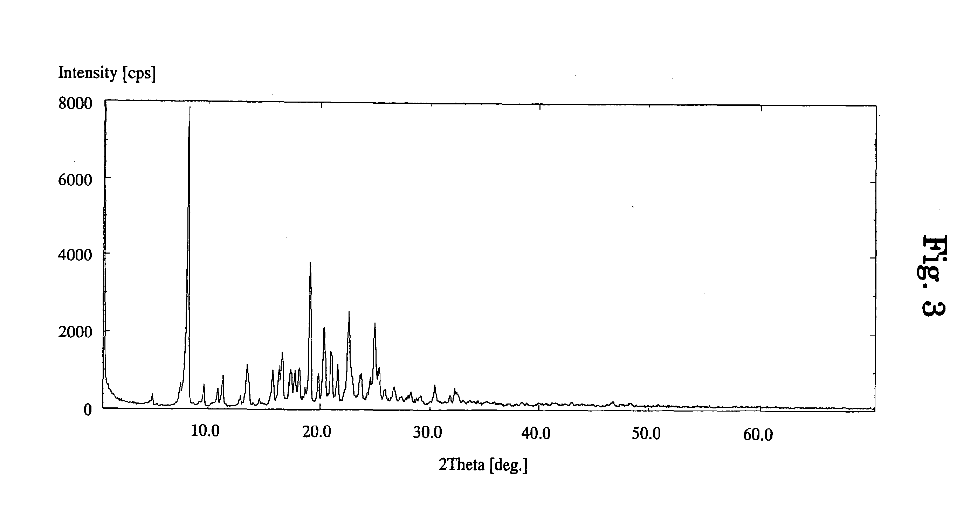

The crystal of the trimethanesulfonate of the compound (I) of the present invention may be a crystal showing main d spacing peaks at 11, 4.6, 4.4, 3.9 and 3.6 Å in a powder X-ray diffraction pattern obtained by irradiation with Cu Kα rays which is shown in Figure 3, for example. Here, the main peak is a peak having a relative intensity of 25 or more when a peak of d spacing at 11 Å has an intensity of 100.

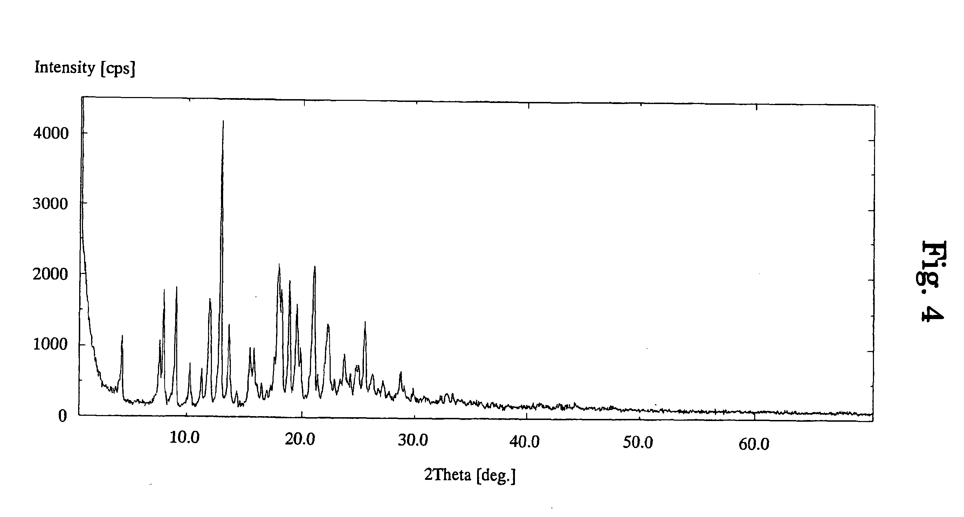

The crystal of the di-p-toluenesulfonate of the compound (I) of the present invention may be a crystal showing main d spacing peaks at 6.8, 4.9, 4.7 and 4.2 Å in a powder X-ray diffraction pattern obtained by irradiation with Cu Kα rays which is shown in Figure 4, for example. Here, the main peak is a peak having a relative intensity of 45 or more when a peak of d spacing at 6.8 Å has an intensity of 100.

In the present invention, heart disease includes angina pectoris. Heart disease, arteriosclerosis or nephropathy includes heart disease, arteriosclerosis or nephropathy caused by hypertension, respectively. Hypertension includes hypertension caused by heart disease, arteriosclerosis or nephropathy.

EFFECTS OF THE INVENTION

The specific acid addition salt of 2-amino-1,4-dihydro-6-methyl-4-(3-nitrophenyl)-3,5-pyridinedicarboxylic acid 3-(1-diphenylmethylazetidin-3-yl) ester 5-isopropyl ester of the present invention has excellent calcium antagonistic effect, hypotensive effect, vasodilative effect, cardioprotective effect, antiarteriosclerotic effect, diuretic effect, nephropathy inhibitory effect and lipid peroxide generation inhibitory effect and is excellent as a pharmaceutical compound in terms of properties such as physicochemical properties, thermal stability, storage and handling stability, residual solvent ratio, hygroscopicity, deliquescence, solubility, pharmacological properties, pharmacokinetic properties, oral absorbability, concentration in blood, bioavailability, pharmacokinetics, safety and toxicity. Therefore, the acid addition salt is useful as a medicine, preferably a medicine for treating or preventing hypertension, heart disease, arteriosclerosis or nephropathy, more preferably a medicine for treating or preventing hypertension or heart disease, and most preferably a medicine for treating or preventing hypertension. Further, the specific acid addition salt of the compound (I) of the present invention may have excellent properties in that the concentration in blood varies only slightly according to a change in intragastric pH and is difficult to be affected by the diet. Therefore, the acid addition salt is useful as a medicine for a warm-blooded animal, and preferably as a medicine for a human.

BEST MODE FOR CARRYING OUT THE INVENTION

In the present invention, the acid addition salt of the compound (I) can be produced by the following method including:
(Step 1) dissolving the compound (I) in an inert solvent or water-containing inert solvent and adding an acid or an aqueous solution or an inert solvent solution of an acid dropwise to the solution;
(Step 2) stirring the mixture at a certain temperature (preferably at room temperature) for a certain period of time; and
(Step 3) collecting the formed solid by filtration and drying the solid.
As necessary, it is possible to carry out before or after Step 2 one or more steps selected from the group consisting of the following steps:
(Step 4-1) adding seed crystals;
(Step 4-2) evaporating part of the solvent;
(Step 4-3) adding a poor solvent (an inert solvent in which the acid addition salt is insoluble); and
(Step 4-4) initiating or promoting precipitation of the crystals by providing mechanical stimulation such as ultrasonic stimulation or abrasion on the surface of the reaction vessel.
In Steps 1 and 2, water is preferably present or a hydrate of an acid is preferably used. In Step 1, an aqueous solution of an acid is preferably added dropwise to a solution of the compound (I) in an inert solvent.

The compound (I) used in the above production method can be produced according to the method described in Example 1 of JP 3-31715A (U.S. Patent No. 4,772,596). The compound (I) may be used as any of an isolated and purified product, a solid state crude reaction product and a solution of a crude reaction product.

The inert solvent used is not particularly limited insofar as it does not inhibit the reaction and allows the starting material to be dissolved therein to a certain extent. Examples of the solvent may include aliphatic hydrocarbons such as hexane, pentane, petroleum ether and cyclohexane; aromatic hydrocarbons such as benzene, toluene and xylene; halogenated hydrocarbons such as methylene chloride, chloroform, carbon tetrachloride, dichloroethane, chlorobenzene and dichlorobenzene; ethers such as diethyl ether, diisopropyl ether, dibutyl ether, butyl methyl ether, sec-butyl methyl ether, tert-butyl methyl ether, tetrahydrofuran, dioxane, dimethoxyethane and diethylene glycol dimethyl ether; ketones such as acetone, methyl ethyl ketone, methyl isobutyl ketone and cyclohexanone; esters such as ethyl acetate, propyl acetate and butyl acetate; nitriles such as acetonitrile, propionitrile, butyronitrile and isobutyronitrile; alcohols such as methanol, ethanol, 1-propanol, 2-propanol, 1-butanol, 2-butanol, 2-methyl-1-propanol and 2-methyl-2-propanol; amides such as formamide, dimethylformamide, dimethylacetamide, N-methyl-2-pyrrolidone and hexamethylphosphoric triamide; water; and mixtures thereof. Preferable examples of the solvent include aromatic hydrocarbons, ethers, ketones, esters, alcohols, water and mixtures thereof, more preferably toluene, tert-butyl methyl ether, acetone, ethyl acetate, methanol, ethanol, 1-propanol, 2-propanol and mixtures of these solvents and water, still more preferably toluene, acetone, ethyl acetate, methanol, ethanol and mixtures of these solvents and water. The inert solvent used for producing the dihydrobromide of the compound (I) is preferably acetone, ethyl acetate, methanol, ethanol or a mixture of these solvents and water, more preferably acetone, methanol or a mixture of these solvents and water, and most preferably methanol or a mixture of methanol and water. The inert solvent used for producing the trimethanesulfonate of the compound (I) is preferably toluene or ethyl acetate, and most preferably ethyl acetate. A suitable amount of water is preferably present in the reaction solution.

When the acid to be used is a monovalent acid, the amount of the acid used may be 0.4 to 10 mol per mol of the compound (I), for example, and is preferably 0.6 to 6 mol, and more preferably 0.8 to 5 mol.

When the acid to be used is a divalent acid, the amount of the acid used may be 0.2 to 10 mol per mol of the compound (I), for example, and is preferably 0.3 to 6 mol, and more preferably 0.4 to 4 mol.

When the acid to be used is a trivalent acid, the amount of the acid used may be 0.1 to 10 mol per mol of the compound (I), for example, and is preferably 0.2 to 6 mol, and more preferably 0.3 to 4 mol.

The concentration of the acid to be used in the aqueous solution or inert solvent solution may be 0.1 mol/l to saturation, for example, and is preferably 1 to 20 mol/l, and more preferably 3 to 15 mol/l.

The reaction temperature is usually -20°C to 150°C, preferably 0°C to 100°C, and more preferably 10°C to 60°C.

The reaction time varies according to the acid to be used, the solvent to be used, the reaction temperature or the like and is usually 5 minutes to 24 hours, preferably 10 minutes to 12 hours, and more preferably 20 minutes to 6 hours.

The formed solid may be isolated by filtration, centrifugation or a gradient method, for example. The isolated solid may be washed with an inert solvent (preferably an inert solvent used in the reaction) as necessary.

The isolated solid may be dried under reduced pressure usually at 20°C to 80°C, and preferably 30°C to 60°C. The drying time is usually a time until the weight becomes almost unchanged, and is preferably 30 minutes to 12 hours, and more preferably 1 to 6 hours. The solid may be dried in the presence of a drying agent such as a silica gel and/or calcium chloride as necessary.

The above reaction conditions are preferably conditions where hydrolysis reaction of two ester groups in the compound (I) does not occur.

The acid addition salt of the compound (I) of the present invention used as a medicine can be administered as is (as a bulk powder). Alternatively, the acid addition salt can be administered orally as a preparation such as tablets, capsules, granules, powder or syrup produced by mixing with an appropriate pharmacologically acceptable excipient or diluent, for example, or parenterally as a preparation such as an injection or suppository similarly produced (preferably orally).

These preparations are produced by a known method using additives such as an excipient, a lubricant, a binder, a disintegrator, an emulsifier, a stabilizer, a corrigent and/or a diluent.

The excipient may be an organic excipient or an inorganic excipient, for example. Examples of the organic excipient may include sugar derivatives such as lactose, sucrose, glucose, mannitol and sorbitol; starch derivatives such as corn starch, potato starch, α-starch and dextrin; cellulose derivatives such as crystalline cellulose; Gum Arabic; dextran; and pullulan. Examples of the inorganic excipient may include light silicic anhydride; and sulfates such as calcium sulfate.

Examples of the lubricant may include stearic acid; stearic acid metal salts such as calcium stearate and magnesium stearate; talc; colloidal silica; waxes such as beeswax and spermaceti; boric acid; adipic acid; sulfates such as sodium sulfate; glycol; fumaric acid; sodium benzoate; D,L-leucine; lauryl sulfates such as sodium lauryl sulfate and magnesium lauryl sulfate; silicic acids such as silicic anhydride and silicic acid hydrate; and the starch derivatives for the aforementioned excipient.

Examples of the binder may include hydroxypropyl cellulose, hydroxypropylmethyl cellulose, polyvinylpyrrolidone, polyethylene glycol and the compounds shown for the aforementioned excipient.

Examples of the disintegrator include cellulose derivatives such as low-substituted hydroxypropyl cellulose, carboxymethyl cellulose, calcium carboxymethyl cellulose and internally crosslinked sodium carboxymethyl cellulose; crosslinked polyvinylpyrrolidone; and chemically modified starches and celluloses such as carboxymethyl starch and sodium carboxymethyl starch.

Examples of the emulsifier may include colloidal clays such as bentonite and bee gum; anionic surfactants such as sodium lauryl sulfate and calcium stearate; cationic surfactants such as benzalkonium chloride; and nonionic surfactants such as polyoxyethylene alkyl ether, polyoxyethylene sorbitan fatty acid ester and sucrose fatty acid ester.

Examples of the stabilizer may include p-hydroxybenzoic acid esters such as methylparaben and propyl-paraben; alcohols such as chlorobutanol, benzyl alcohol and phenylethyl alcohol; benzalkonium chloride; phenols such as phenol and cresol; thimerosal; dehydroacetic acid; and sorbic acid.

Examples of the corrigent may include sweeteners such as sodium saccharin and aspartame; acidulants such as citric acid, malic acid and tartaric acid; and flavors such as menthol, lemon extract and orange extract.

The diluent may be a compound usually used as a diluent. Examples of the diluent may include lactose, mannitol, glucose, sucrose, calcium sulfate, hydroxypropyl cellulose, microcrystalline cellulose, water, ethanol, polyethylene glycol, propylene glycol, glycerol, starch, polyvinylpyrrolidones and mixtures thereof.

The dose of the acid addition salt of the compound (I) of the present invention may vary according to the conditions such as the symptom, age and body weight of the patient. The acid addition salt can be orally administered at 0.005 mg/kg (preferably 0.02 mg/kg) as the lower limit to 20 mg/kg (preferably 10 mg/kg) as the upper limit, or parenterally administered at 0.0005 mg/kg (preferably 0.002 mg/kg) as the lower limit to 20 mg/kg (preferably 10 mg/kg) as the upper limit to an adult in one to six times per day in response to the symptom.

### EXAMPLES

The present invention will be described in more detail below with reference to Examples, Reference Examples, Test Example and Preparation Examples; however, the scope of the present invention is not limited thereto. In the following Examples, 2-amino-1,4-dihydro-6-methyl-4-(3-nitrophenyl)-3,5-pyridinedicarboxylic acid 3-(1-diphenylmethylazetidin-3-yl) ester 5-isopropyl ester can be produced according to the method described in Example 1 of JP 3-31715A (U.S. Patent No. 4,772,596).

### (Example 1) 2-Amino-1,4-dihydro-6-methyl-4-(3-nitrophenyl)-3,5-pyridinedicarboxylic acid 3-(1-diphenylmethylazetidin-3-yl) ester 5-isopropyl ester dihydrobromide

To a solution of 2.91 g (5.00 mmol) of 2-amino-1,4-dihydro-6-methyl-4-(3-nitrophenyl)-3,5-pyridinedicarboxylic acid 3-(1-diphenylmethylazetidin-3-yl) ester 5-isopropyl ester in 12 ml of methanol was added dropwise 1.27 ml (11.0 mmol) of 47 wt% hydrobromic acid at 25°C over 30 minutes. After completion of the dropwise addition, the reaction solution was further stirred for 30 minutes. The formed crude crystals were collected by filtration and then washed with 12 ml of methanol and dried under reduced pressure at 50°C for two hours to give 2.90 g (78%) of the title compound as a white powder.
¹H-NMR spectrum (DMSO-d₆, δppm): 1.00(d;J=6Hz,3H), 1.19(d;J=6Hz,3H), 2.29(s,3H), 4.01-4.38(m,4H), 4.74-4.92(m,2H), 4.96-5.23(m,1H), 5.78-6.11(m,1H), 6.85(brs,2H), 7.37-7.77(m,12H), 7.94-8.09(m,2H), 8.98(brs,1H), 11.17-11.77(m,1H).

The powder X-ray diffraction pattern of this compound is shown in Figure 1. This compound had specific peaks in the X-ray diffraction pattern and was a crystalline solid.

This compound can also be produced using tert-butyl methyl ether, acetone, ethyl acetate, ethanol, 1-propanol or 2-propanol as a solvent. The solvent used for producing this compound is preferably acetone, ethyl acetate, methanol or ethanol, more preferably acetone or methanol, and most preferably methanol.

### (Example 2) 2-Amino-1,4-dihydro-6-methyl-4-(3-nitrophenyl)-3,5-pyridinedicarboxylic acid 3-(1-diphenylmethylazetidin-3-yl) ester 5-isopropyl ester monocitrate

To a solution of 3.24 g (5.56 mmol) of 2-amino-1,4-dihydro-6-methyl-4-(3-nitrophenyl)-3,5-pyridinedicarboxylic acid 3-(1-diphenylmethylazetidin-3-yl) ester 5-isopropyl ester in 11.1 ml of acetone was added dropwise a solution of 1.11 g (5.83 mmol) of citric acid in 11.1 ml of acetone at 25°C over 30 minutes. After completion of the dropwise addition, the reaction solution was further stirred for 40 minutes. The formed crude crystals were collected by filtration and then washed with 5 ml of acetone and dried under reduced pressure at 50°C for two hours to give 3.42 g (79%) of the title compound as a yellow powder.
¹H-NMR spectrum (DMSO-d₆, δppm): 0.99(d;J=6Hz,3H), 1.18(d;J=6Hz,3H), 2.27(s,3H), 2.30-2.42(m,1H), 2.64(d;J=15Hz,2H), 2.75(d;J=15Hz,2H), 2.86-2.97(m,1H), 3.28-3.40(m,1H), 3.49-3.58(m,1H), 4.21(s,1H), 4.72-4.91(m,3H), 6.75(brs,2H), 7.12-7.41(m,10H), 7.54-7.67(m,2H), 8.02-8.12(m,2H), 8.80(brs,1H), 12.34(brs,1H).

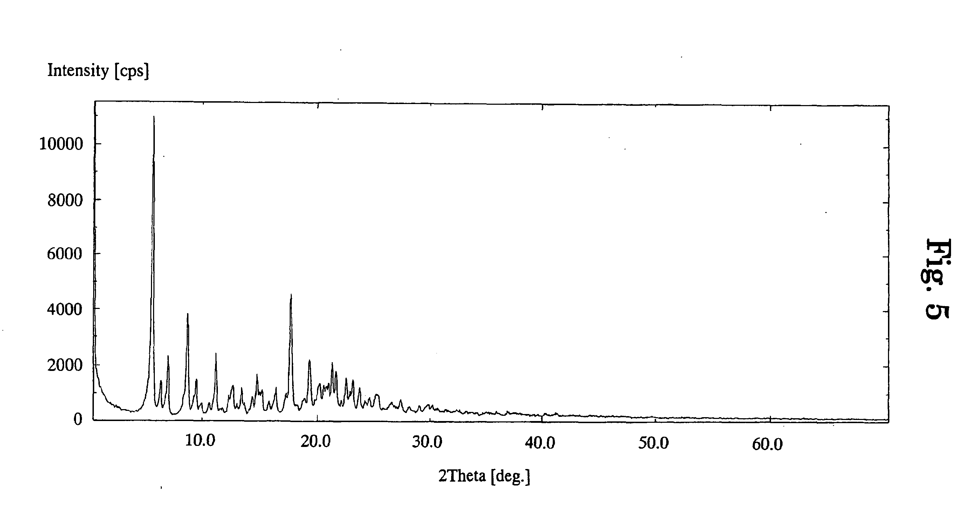

The powder X-ray diffraction pattern of this compound is shown in Figure 5. This compound had specific peaks in the X-ray diffraction pattern and was a crystalline solid.

### (Example 3) 2-Amino-1,4-dihydro-6-methyl-4-(3-nitrophenyl)-3,5-pyridinedicarboxylic acid 3-(1-diphenylmethylazetidin-3-yl) ester 5-isopropyl ester dimethanesulfonate

To a solution of 4.66 g (8.00 mmol) of 2-amino-1,4-dihydro-6-methyl-4-(3-nitrophenyl)-3,5-pyridinedicarboxylic acid 3-(1-diphenylmethylazetidin-3-yl) ester 5-isopropyl ester in 32 ml of ethyl acetate was added dropwise 1.04 ml (16.0 mmol) of methanesulfonic acid at 25°C over 30 minutes. After completion of the dropwise addition, the reaction solution was further stirred for 30 minutes. The formed crude crystals were collected by filtration and then washed with 20 ml of ethyl acetate and dried under reduced pressure at 40°C for two hours to give 6.01 g (97%) of the title compound as a white powder.
¹H-NMR spectrum (DMSO-d₆, δppm): 1.00(d;J=6Hz,3H), 1.18(d;J=6Hz,3H), 2.28(s,3H), 2.38(s,6H), 4.03-4.42(m,4H), 4.73-4.89(m,2H), 4.96-5.18(m,1H), 5.73-6.01(m,1H), 6.85(brs,2H), 7.37-7.67(m,12H), 7.93-8.04(m,2H), 8.96(brs,1H), 11.01-11.42(m,1H).

The powder X-ray diffraction pattern of this compound is shown in Figure 2. This compound had specific peaks in the X-ray diffraction pattern and was a crystalline solid.

This compound can also be produced using tert-butyl methyl ether, methanol, ethanol or 2-propanol as a solvent.

### (Example 4) 2-Amino-1,4-dihydro-6-methyl-4-(3-nitrophenyl)-3,5-pyridinedicarboxylic acid 3-(1-diphenylmethylazetidin-3-yl) ester 5-isopropyl ester trimethanesulfonate

To a solution of 4.66 g (8.00 mmol) of 2-amino-1,4-dihydro-6-methyl-4-(3-nitrophenyl)-3,5-pyridinedicarboxylic acid 3-(1-diphenylmethylazetidin-3-yl) ester 5-isopropyl ester in 32 ml of ethyl acetate was added dropwise 1.71 ml (26.4 mmol) of methanesulfonic acid at 25°C over 30 minutes. After completion of the dropwise addition, the reaction solution was further stirred for 30 minutes. The formed crude crystals were collected by filtration and then washed with 20 ml of ethyl acetate and dried under reduced pressure at 40°C for two hours to give 6.78 g (97%) of the title compound as a white powder.
¹H-NMR spectrum (DMSO-d₆, δppm): 1.00(d;J=6Hz,3H), 1.19(d;J=6Hz,3H), 2.28(s,3H), 2.40(s,9H), 4.02-4.42(m,4H), 4.75-4.88(m,2H), 4.98-5.18(m,1H), 5.73-6.10(m,1H), 6.84(brs,2H), 7.35-7.69(m,12H), 7.91-8.07(m,2H), 8.97(brs,1H), 11.01-11.45(m,1H).

The powder X-ray diffraction pattern of this compound is shown in Figure 3. This compound had specific peaks in the X-ray diffraction pattern and was a crystalline solid.

This compound can also be produced using toluene as a solvent.

### (Example 5) 2-Amino-1,4-dihydro-6-methyl-4-(3-nitrophenyl)-3,5-pyridinedicarboxylic acid 3-(1-diphenylmethylazetidin-3-yl) ester 5-isopropyl ester dibenzenesulfonate

To a solution of 4.66 g (8.00 mmol) of 2-amino-1,4-dihydro-6-methyl-4-(3-nitrophenyl)-3,5-pyridinedicarboxylic acid 3-(1-diphenylmethylazetidin-3-yl) ester 5-isopropyl ester in 32 ml of ethyl acetate was added dropwise a solution of 4.23 g (24.0 mmol) of benzenesulfonic acid monohydrate in 15 ml of ethyl acetate at 25°C over 30 minutes. After completion of the dropwise addition, the reaction solution was further stirred for 30 minutes. The formed crude crystals were collected by filtration and then washed with 20 ml of ethyl acetate and dried under reduced pressure at 40°C for two hours to give 7.13 g (99%) of the title compound as a white powder.
¹H-NMR spectrum (DMSO-d₆, δppm): 1.00(d;J=6Hz,3H), 1.19(dd;J=6Hz,2Hz,3H), 2.28(s,3H), 3.98-4.44(m,4H), 4.72-4.96(m,2H), 4.94-5.18(m,1H), 5.72-6.00(m,1H), 6.84(brs,2H), 7.26-7.67(m,22H), 7.93-8.03(m,2H), 8.92(brs,1H), 10.88-11.38(m,1H).

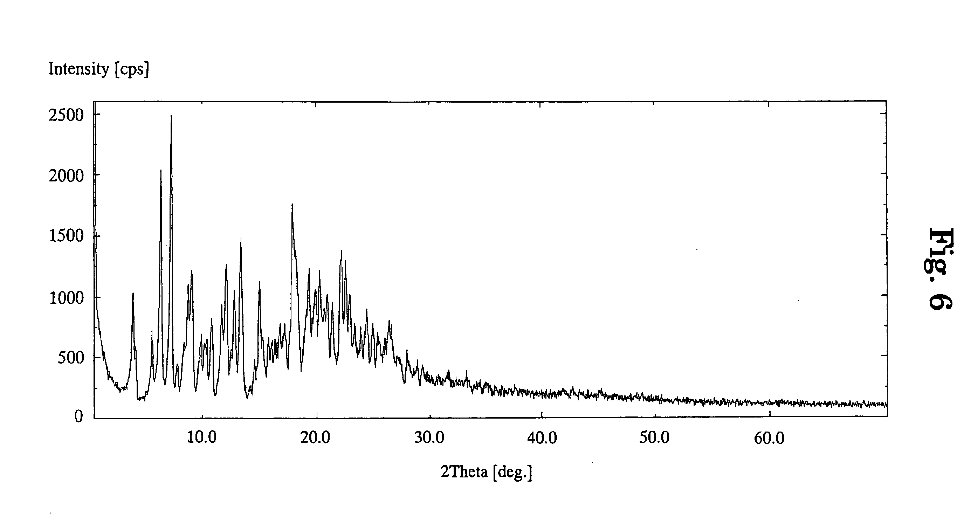

The powder X-ray diffraction pattern of this compound is shown in Figure 6. This compound had specific peaks in the X-ray diffraction pattern and was a crystalline solid.

### (Example 6) 2-Amino-1,4-dihydro-6-methyl-4-(3-nitrophenyl)-3,5-pyridinedicarboxylic acid 3-(1-diphenylmethylazetidin-3-yl) ester 5-isopropyl ester di-p-toluenesulfonate

To a solution of 4.66 g (8.00 mmol) of 2-amino-1,4-dihydro-6-methyl-4-(3-nitrophenyl)-3,5-pyridinedicarboxylic acid 3-(1-diphenylmethylazetidin-3-yl) ester 5-isopropyl ester in 32 ml of ethyl acetate was added dropwise a solution of 4.57 g (24.0 mmol) of p-toluenesulfonic acid monohydrate in 20 ml of ethyl acetate at 25°C over 30 minutes. After completion of the dropwise addition, the reaction solution was further stirred for 30 minutes. The formed crude crystals were collected by filtration and then washed with 20 ml of ethyl acetate and dried under reduced pressure at 40°C for two hours to give 7.65 g (quant.) of the title compound as a white powder.
¹H-NMR spectrum (DMSO-d₆, δppm): 1.00(d;J=6Hz,3H), 1.18(dd;J=6Hz,2Hz,3H), 2.28(s,3H), 2.29(s,6H), 4.08-4.43(m,4H), 4.73-4.90(m,2H), 4.96-5.19(m,1H), 5.74-6.00(m,1H), 6.85(brs,2H), 7.10-7.16(m,4H), 7.38-7.66(m,16H), 7.94-8.05(m,2H), 8.92(brs,1H), 10.93-11.42(m,1H).

The powder X-ray diffraction pattern of this compound is shown in Figure 4. This compound had specific peaks in the X-ray diffraction pattern and was a crystalline solid.

### (Example 7) 2-Amino-1,4-dihydro-6-methyl-4-(3-nitrophenyl)-3,5-pyridinedicarboxylic acid 3-(1-diphenylmethylazetidin-3-yl) ester 5-isopropyl ester dinaphthalenesulfonate

To a solution of 4.66 g (8.00 mmol) of 2-amino-1,4-dihydro-6-methyl-4-(3-nitrophenyl)-3,5-pyridinedicarboxylic acid 3-(1-diphenylmethylazetidin-3-yl) ester 5-isopropyl ester in 32 ml of ethyl acetate was added dropwise a solution of 5.97 g (26.4 mmol) of 2-naphthalenesulfonic acid monohydrate in 120 ml of ethyl acetate at 25°C over 30 minutes. After completion of the dropwise addition, the reaction solution was further stirred for 30 minutes. The reaction solution was allowed to stand at 2°C for four days to precipitate the solid. The precipitated crude solid was collected by filtration and then washed with 100 ml of ethyl acetate and dried under reduced pressure at 45°C for three hours to give 7.72 g (97%) of the title compound as a white powder.
¹H-NMR spectrum (DMSO-d₆, δppm): 1.00(d;J=6Hz,3H), 1.18(dd;J=6Hz,2Hz,3H), 2.28(s,3H), 4.06-4.42(m,4H), 4.74-4.90(m,2H), 4.96-5.18(m,1H), 5.73-6.00(m,1H), 6.84(brs,2H), 7.37-7.57(m,15H), 7.60-7.66(m,1H), 7.70-7.76(m,2H), 7.85-8.04(m,8H), 8.14-8.18(m,2H), 8.92(brs,1H), 10.88-11.37(m,1H).

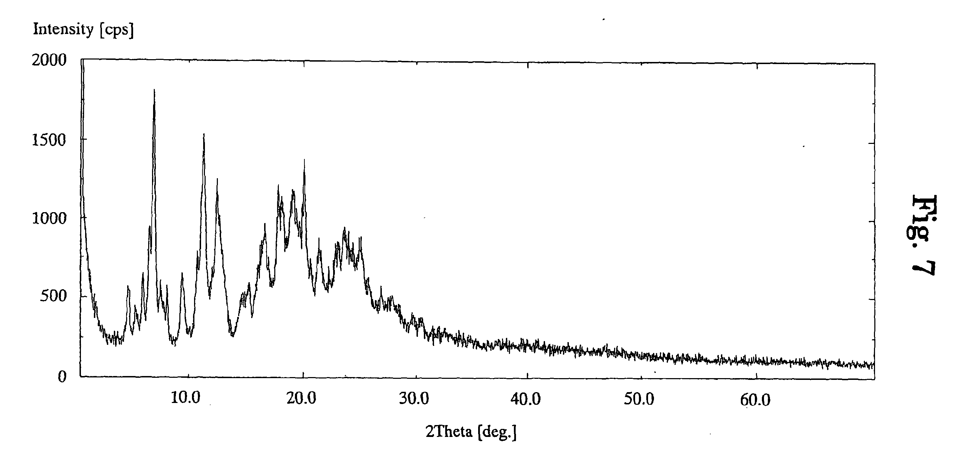

The powder X-ray diffraction pattern of this compound is shown in Figure 7. This compound had specific peaks in the X-ray diffraction pattern and was a crystalline solid.

### (Comparative Example 1) 2-Amino-1,4-dihydro-6-methyl-4-(3-nitrophenyl)-3,5-pyridinedicarboxylic acid 3-(1-diphenylmethylazetidin-3-yl) ester 5-isopropyl ester dihydrochloride (aqueous solution method)

To a solution of 4.66 g (8.00 mmol) of 2-amino-1,4-dihydro-6-methyl-4-(3-nitrophenyl)-3,5-pyridinedicarboxylic acid 3-(1-diphenylmethylazetidin-3-yl) ester 5-isopropyl ester in 32 ml of ethyl acetate was added dropwise 1.49 ml (17.6 mmol) of 36 wt% hydrochloric acid at 25°C over 30 minutes. After completion of the dropwise addition, the reaction solution was further stirred for 30 minutes. The formed crude crystals were collected by filtration and then washed with 10 ml of ethyl acetate and dried under reduced pressure at 50°C for one hour to give 5.26 g (92%) of the title compound as a white powder.
¹H-NMR spectrum (DMSO-d₆, δppm): 0.99(d;J=6Hz,3H), 1.19(dd;J=6Hz,3Hz,3H), 2.29(s,3H), 3.90-4.28(m,4H), 4.70-5.32(m,3H), 5.68-6.03(m,1H), 6.93(brs,2H), 7.32-7.77(m,12H), 7.91-8.06(m,2H), 9.21-9.38(m,1H), 12.58-12.91(m,1H).

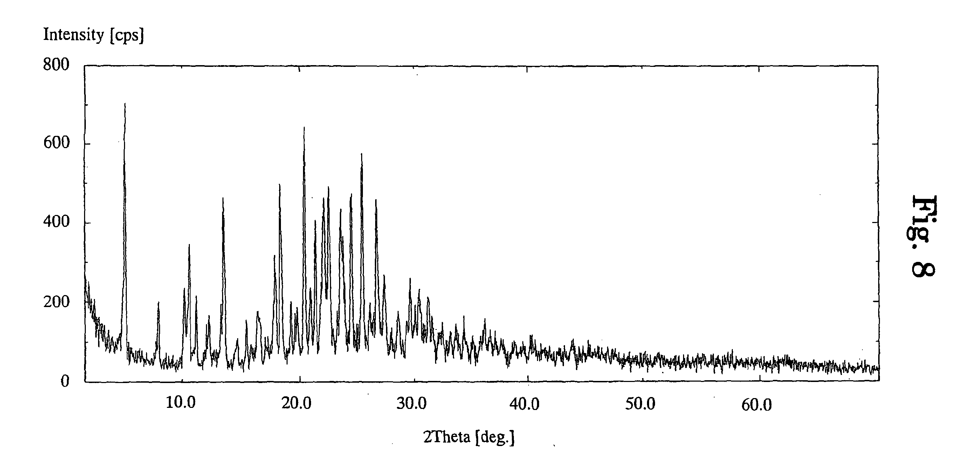

The powder X-ray diffraction pattern of this compound is shown in Figure 8. This compound obtained by the aqueous solution method had specific peaks in the X-ray diffraction pattern and was a crystalline solid.

### (Comparative Example 2) 2-Amino-1,4-dihydro-6-methyl-4-(3-nitrophenyl)-3,5-pyridinedicarboxylic acid 3-(1-diphenylmethylazetidin-3-yl) ester 5-isopropyl ester dihydrochloride (gas method)

The title compound was produced according to the method described in Example 1 of JP 3-31715A (U.S. Patent No. 4,772,596).

A solution of 2.91 g (5.00 mmol) of 2-amino-1,4-dihydro-6-methyl-4-(3-nitrophenyl)-3,5-pyridinedicarboxylic acid 3-(1-diphenylmethylazetidin-3-yl) ester 5-isopropyl ester in 66.7 ml of chloroform was bubbled with hydrochloric acid gas with stirring at 25°C for five minutes. The reaction solution was concentrated under reduced pressure, and the resulting solid was dried under reduced pressure at 50°C for two hours to give 3.65 g (quant.) of the title compound as a pale yellow solid.

The ¹H-NMR spectrum of this compound was the same as that of the compound of Comparative Example 1.

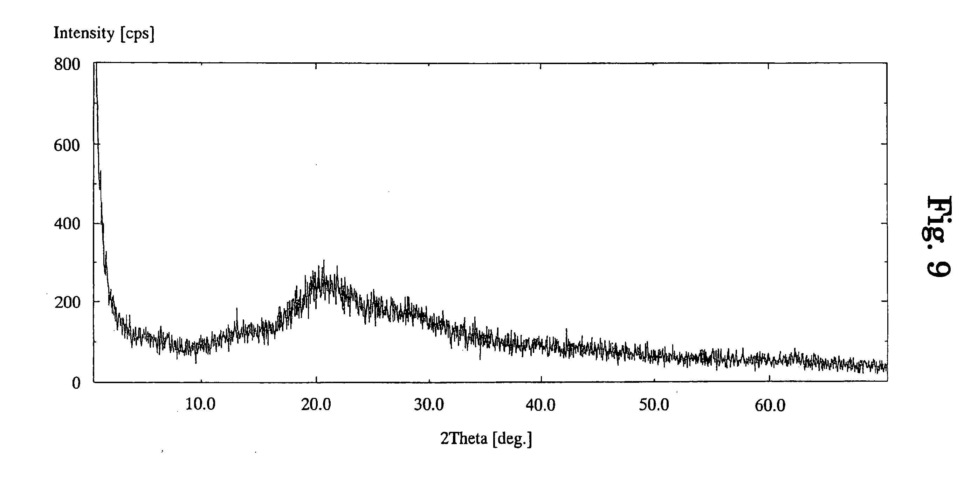

The powder X-ray diffraction pattern of this compound is shown in Figure 9. This compound obtained by the gas method did not have a specific peak in the X-ray diffraction pattern and was an amorphous solid.

The results of Comparative Examples 1 and 2 showed that a production method using an aqueous acid solution is useful for obtaining the crystalline acid addition salt of the compound (I).

### (Comparative Example 3) 2-Amino-1,4-dihydro-6-methyl-4-(3-nitrophenyl)-3,5-pyridinedicarboxylic acid 3-(1-diphenylmethylazetidin-3-yl) ester 5-isopropyl ester monosulfate

To a solution of 6.99 g (12.0 mmol) of 2-amino-1,4-dihydro-6-methyl-4-(3-nitrophenyl)-3,5-pyridinedicarboxylic acid 3-(1-diphenylmethylazetidin-3-yl) ester 5-isopropyl ester in 24 ml of acetone was added dropwise 0.70 ml (12.6 mmol) of 96% sulfuric acid at 25°C over 30 minutes. After completion of the dropwise addition, the reaction solution was further stirred for 20 minutes. The formed yellow viscous solid was left to stand for two hours. Then, the solidified solid was washed with 30 ml of acetone and dried under reduced pressure at 45°C for two hours to give 7.55 g (92%) of the title compound as a yellowish white powder.
¹H-NMR spectrum (DMSO-d₆, δppm): 1.00(d;J=6Hz,3H), 1.18(d;J=6Hz,3H), 2.28(s,3H), 4.00-4.43(m,4H), 4.72-4.90(m,2H), 4.96-5.18(m,1H), 5.64-6.02(m,1H), 6.85(brs,2H), 7.33-7.70(m,12H), 7.92-8.08(m,2H), 8.93(brs,1H), 10.93-11.41(m,1H).

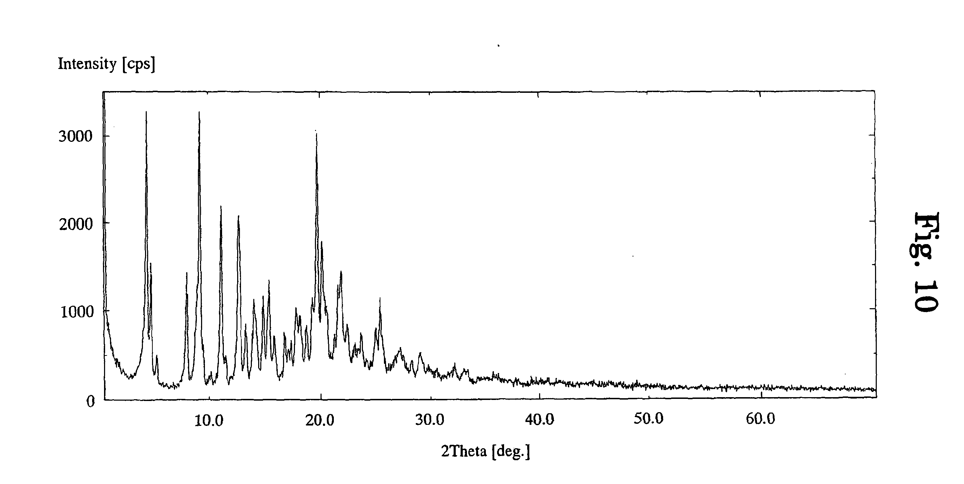

The powder X-ray diffraction pattern of this compound is shown in Figure 10. This compound had specific peaks in the X-ray diffraction pattern and was a crystalline solid. However, in the above production step, it was necessary to leave the yellow viscous solid to stand and solidify the solid in order to form crystals. This compound had crystallinity lower than that of the specific acid addition salt of the compound (I) of the present invention.

### (Comparative Example 4) 2-Amino-1,4-dihydro-6-methyl-4-(3-nitrophenyl)-3,5-pyridinedicarboxylic acid 3-(1-diphenylmethylazetidin-3-yl) ester 5-isopropyl ester hemifumarate

To a solution of 5.00 g (8.58 mmol) of 2-amino-1,4-dihydro-6-methyl-4-(3-nitrophenyl)-3,5-pyridinedicarboxylic acid 3-(1-diphenylmethylazetidin-3-yl) ester 5-isopropyl ester in 15 ml of ethyl acetate was added dropwise a solution of 997 mg (8.59 mmol) of fumaric acid in 24 ml of tetrahydrofuran at 25°C over 35 minutes. After completion of the dropwise addition, the reaction solution was further stirred for 15 minutes and then 78 ml of diisopropyl ether was added. The formed solid was collected by filtration and then washed with 5 ml of ethyl acetate and dried under reduced pressure at 45°C for two hours to give 5.14 g (93%) of the title compound as a yellow powder.
¹H-NMR spectrum (DMSO-d₆, δppm): 0.99(d;J=6Hz,3H), 1.18(d;J=6Hz,3H), 2.22-2.39(m,4H), 2.85-2.92(m,1H), 3.22-3.50(m,2H), 4.18(s,1H), 4.72-4.91(m,3H), 6.63(s,1H), 6.77(brs,2H), 7.13-7.41(m,10H), 7.55-7.66(m,2H), 8.03-8.12(m,2H), 8.83(brs,1H), 13.12(brs,1H)

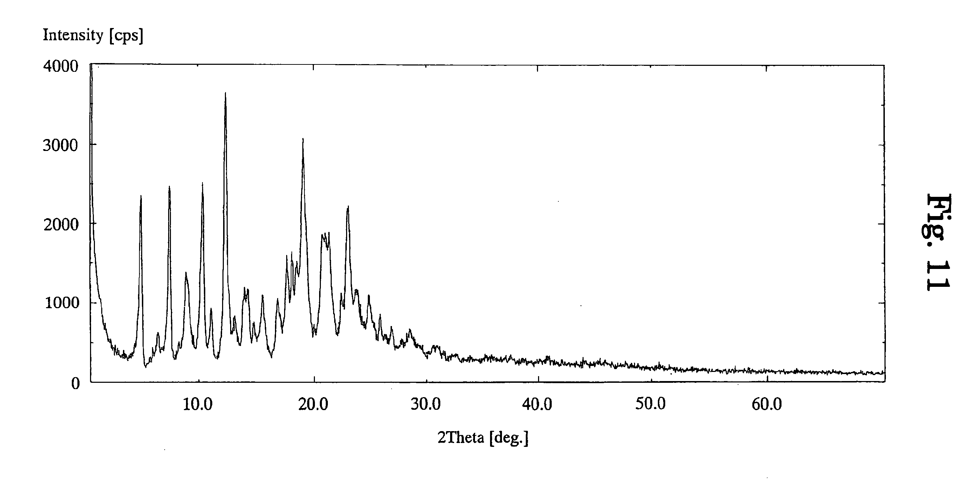

The powder X-ray diffraction pattern of this compound is shown in Figure 11. This compound had specific peaks in the X-ray diffraction pattern and was a crystalline solid. However, in the above production step, it was necessary to add a poor solvent (diisopropyl ether) in order to form crystals. This compound had crystallinity lower than that of the specific acid addition salt of the compound (I) of the present invention.

### (Test Example 1) Thermal stability test

The test compound was placed in a glass bottle and allowed to stand under the following conditions (1) and (2). After a certain period of time had elapsed, the residual rate of the active ingredient [compound (I)] in the test compound was measured by high performance liquid chromatography.

(1): Closed state, 60°C;
(2): Non-closed state, 40°C, humidity 75%.

The measurement conditions in high performance liquid chromatography are as follows.

Column: L-column ODS [4.6 mm x 250 mm, manufactured by Chemicals Evaluation and Research Institute, Japan]
Mobile layer: acetonitrile/22 mM potassium dihydrogenphosphate buffer/methanol = 455/350/195 (V/V/V) (adjusted to pH 5.5 by phosphoric acid)
Flow rate: 1 ml/min
Column temperature: 40°C
Detection wavelength: 220 nm
The residual rate (%) was calculated by the following formula.

The residual rate (%) = [1 - (sum of peak area percentages of impurities and decomposed products)] x 100
The results under the above conditions (1) and (2) are shown in Tables 1 and 2, respectively.

**[Table 1] [Condition (1)]**

| Test compound No. | Residual rate of Compound (I) (%) | | | | |
|---|---|---|---|---|---|
| | Time (month): | | | | |
| | | 0 | 1 | 3 | 6 |
| Example 1 | | 99.9 | 99.7 | 99.7 | 99.6 |
| Example 2 | | 98.8 | 96.9 | 95.8 | |
| Example 3 | | 99.6 | 98.7 | 98.5 | |
| Example 4 | | 99.5 | 98.6 | 98.1 | |
| Example 5 | | 99.7 | 98.6 | 96.5 | 93.3 |
| Example 6 | | 99.6 | 98.8 | 98.3 | 97.3 |
| Example 7 | | 99.4 | 97.3 | 95.4 | 93.0 |
| Comparative Example 1 | | 98.1 | 60.0 | 58.9 | |
| Comparative Example 2 | | 97.3 | 10.3 | | |
| Comparative Example 3 | | 99.5 | 96.9 | 94.9 | 92.8 |
| Comparative Example 4 | | 99.7 | 96.7 | 93.0 | |

As is clear from the results in Table 1, the compound of Comparative Example 2 that was an amorphous solid had extremely low stability, and the compound of Comparative Example 1 had extremely low stability although the compound was a crystalline solid. The compounds of Comparative Examples 3 and 4 did not have high stability although the compounds were crystalline solids. In contrast, the compounds of Examples 1 to 7 of the present invention were crystalline solids and had excellent stability. The compounds of Examples 2, 5 and 7 of the present invention were equal in stability but were superior in crystallinity to the compounds of Comparative Examples 3 and 4 (see the above Comparative Examples 3 and 4).

**[Table 2] [Condition (2)]**

| Test compound No. | Residual rate of Compound (I) (%) | | | | |
|---|---|---|---|---|---|
| | Time (month): | | | | |
| | | 0 | 1 | 3 | 6 |
| Example 1 | | 99.9 | 99.5 | 99.1 | 98.9 |
| Example 4 | | 99.5 | 99.1 | 98.8 | |
| Example 6 | | 99.6 | 97.5 | 96.4 | 94.5 |
| Comparative Example 1 | | 98.1 | 62.9 | 59.0 | |
| Comparative Example 2 | | 97.3 | 11.3 | | |
| Comparative Example 3 | | 99.5 | 93.9 | 88.9 | 84.8 |
| Comparative Example 4 | | 99.7 | 94.8 | 91.3 | |

As is clear from the results in Table 2, the compound of Comparative Example 2 that was an amorphous solid had extremely low stability, and the compounds of Comparative Examples 1, 3 and 4 had low stability although the compounds were crystalline solids. In contrast, the compounds of Examples 1, 4 and 6 of the present invention were crystalline solids and had excellent stability.

The results in Tables 1 and 2 show that not all acid addition salts of the compound (I) that can be obtained as crystalline solids have excellent thermal stability and the specific acid addition salts of the compound (I) of the present invention have thermal stability superior to those of other acid addition salts.

### (Test Example 2) Absorbability test in dog

### (1) Method

Tetragastrin (6 µg/kg) was intramuscularly injected to the posterior region of thigh of a fasted male beagle (body weight: about 10 kg, n = 4) to make the intragastric pH acidic 30 minutes before, immediately before and 30 minutes after administration of the test compound. The test compound was encapsulated in gelatin capsules at a dose of 100 mg/body and orally administered. About 3 ml of blood was collected from the median antebrachial vein using a heparin-treated glass syringe 0.5, 1, 2, 3, 4, 6, 8 and 24 hours after the administration. The collected blood was centrifuged to obtain plasma which was cryopreserved at -20°C until measurement of the concentration of the test compound. One hundred µl of thawed plasma was mixed with 100 µl of 50% methanol, 800 µl of purified water, 100 µl of 0.1 N aqueous hydrochloric acid and 100 µl of an internal standard solution [a solution of the d₇-compound (I) in 50% methanol, 50 ng/ml]. The eluate obtained by solid phase extraction using OASIS HLB was evaporated to dryness and then dissolved in 400 µl of 75% methanol. The solution was analyzed by LC/MS/MS.

A calibration curve was prepared by the same operation as above using 100 µl of dog control plasma without drug administration and 100 µl of a calibration curve standard solution [a solution of the compound (I) in 50% methanol, 0.1-1000 ng/ml].

The LC/MS/MS analysis conditions are shown below. [MS/MS]
System: API4000 LC/MS/MS System (manufactured by Applied Biosystems/MDS SCIEX)
Ion source: TurboIonSpray
Turbo heater gas: Air, 650°C, 70 psi
Nebulizer gas: Air, 70 psi
Curtain gas: N₂, 40
Orifice voltage: Compound (I) 106 V
   d₇-Compound (I) 106 V
Ion spray voltage: 5500 V
Collision gas: N₂, 3
Collision energy: Compound (I) 35 V
   d₇-Compound (I) 35 V
Measurement mode: Positive/MRM
Monitor ion: Compound (I) m/z 583 -> 167
   d₇-Compound (I) m/z 590 -> 167
[HPLC]
System: Agilent 1100 Series (manufactured by Agilent Technologies)
Column: Inertsil ODS-3 5 µm, 2.1 mm I.D. x 150 mm (manufactured by GL Sciences)
Mobile phase: Methanol/water/difluoroacetic acid (700/300/0.75)
Flow rate: 0.15 ml/min
Column temperature: 40°C
Injection amount: 5 µl

### (2) Results

The area under the concentration in plasma-time curve (AUC₀₋₂₄ₕ) and the maximum concentration in plasma (Cmax) as pharmacokinetic parameters indicative of drug absorbability were calculated from the results obtained according to the aforementioned method using the compound of Example 1 and the compound (I) as test compounds. The results are shown in Table 3.

**[Table 3]**

| Test compound No. | AUC₀₋₂₄ₕ [ng.h/ml] | Cmax [ng/ml] |
|---|---|---|
| Example 1 | 2933 | 248 |
| Compound (I) | 1813 | 178 |

The compound of Example 1 of the present invention had bioavailability and concentration in blood (AUC₀₋₂₄ₕ and Cmax) superior to those of the free compound (I).

### (Test Example 3) Elution test

### (1) Method

A test was carried out using one gelatin capsule filled with the test compound (100 mg equivalent/capsule) and 900 ml of the disintegration test first solution (The Japanese Pharmacopoeia Fourteenth Edition, pH 1.2) as a test solution according to the elution test second method (The Japanese Pharmacopoeia Fourteenth Edition, paddle method) at 250 revolutions per minute. A sinker (The Japanese Pharmacopoeia Fourteenth Edition elution test method) was used for preventing flotation of the gelatin capsule. Five, 10, 15, 20, 30, 45, 60, 75, 90, 105 and 120 minutes after the start of the elution test, about 20 ml of the test solution was filtered through a filter having a pore size of 10 µm (manufactured by VARIAN, Full Flow Filters 10 µm) and directly fed into a flow cell (optical path length: 0.5 cm) in a circulating system. The absorbance was measured by a photodiode array spectrophotometer (HP 8452A). The absorbance of the compound (I) in each test solution at the time of collection of the test solution was compared with the absorbance of the following standard solution to measure the concentration of the compound (I) in the test solution.

The standard solution was prepared as follows. About 28 mg of the compound (I) was precisely weighed and added to a 500 ml volumetric flask. Five ml of ethanol (99.5%, special grade reagent) was added to the flask and the compound (I) was dissolved while providing ultrasonic stimulation as necessary. The disintegration test first solution was added to make the volume exactly 500 ml. The resulting solution was provided as the standard solution.

The elution test system is shown below.
Elution test device: DT-600 (manufactured by JASCO)
Ultraviolet visible spectrophotometer: HP 8452A Diode-array UV-Visible Spectrophotometer (manufactured by Hewlett Packard; now Agilent Technologies)
Cell positioner: HP 89075C Multicell Transport (manufactured by Hewlett Packard)
Delivery pump: HP 89092A Multichannel Pump (manufactured by Hewlett Packard)
Elution test software: Hewlett Packard Dissolution Testing Software, Revision 03.01 (manufactured by Hewlett Packard)
Analysis wavelength: 254 to 258 nm
Control wavelength: 322 to 326 nm
Elapsed time: 1 sec
Optical path length of quartz cell: 0.5 cm

### (2) Results

The elution rates calculated from the concentration of the compound (I) in the test solution measured in the above test using the compound of Example 1 and the compound (I) as test compounds are shown in Table 4.

**[Table 4]**

| Test compound No. | Elution rate (%) | | | | | | |
|---|---|---|---|---|---|---|---|
| | Time (min) : | | | | | | |
| | | 5 | 10 | 15 | 20 | 30 | 45 |
| Example 1 | | 1.0 | 1.9 | 3.3 | 5.0 | 9.9 | 23.5 |
| Compound (I) | | 0.5 | 0.7 | 1.0 | 1.3 | 1.8 | 2.5 |
| | | | | | | | |

| Test compound No. | Elution rate (%) | | | | | | |
|---|---|---|---|---|---|---|---|
| | Time (min) : | | | | | | |
| | | 60 | 75 | 90 | 105 | 120 | |
| Example 1 | | 35.5 | 40.8 | 45.1 | 49.6 | 52.6 | |
| Compound (I) | | 3.1 | 3.6 | 4.1 | 4.5 | 5.0 | |

The compound of Example 1 of the present invention had elution properties superior to those of the free compound (I). Therefore, the compound of Example 1 is assumed to have more excellent oral absorbability.

### (Test Example 4) Calcium channel receptor binding test using rat cerebral cortex membrane fraction

A rat cerebral cortex membrane fraction was used as a source of the L-type calcium channel, and ³H-(+)-isradipine was used as a ligand of the L-type calcium channel. The membrane fraction (5.0 mg protein/ml), ³H-(+)-isradipine (0.5 nM) and the test compound were reacted in a tris-(hydroxymethyl)aminomethane hydrochloride (Tris-HCl; 50 mM, pH 7.4) buffer at room temperature for 60 minutes. Then, ³H-(+)-isradipine bound to the membrane fraction was measured using a liquid scintillation counter. The count in the presence of unlabeled nitrendipine (non-specific binding amount) was subtracted from the measured count to calculate the specific binding amount. The relation of the specific binding concentration and the binding inhibition rate for each compound was applied to a logit-log model to calculate the IC₅₀ value (50% inhibitory concentration of specific binding; nM) and the Ki value (dissociation constant; nM).

The compounds of Examples 1 to 7 had a Ki value of 4.8 to 9.2 nM each. The specific acid addition salt of the compound (I) of the present invention has an excellent calcium channel receptor antagonistic effect and is useful as a medicine for treating or preventing hypertension, heart disease, arteriosclerosis or nephropathy.

Test Example 4 may also be carried out using porcine myocardium microsome as a source of the L-type calcium channel.

### (Test Example 5) Hypotensive effect test in hypertensive rat

The test compound was orally administered to a male spontaneously hypertensive rat without anaesthesia, and the blood pressure was measured by telemetry method every five minutes over 24 hours. The test compound was suspended in a 0.5% methylcellulose solution and administered to the rat. The area from the time of administration to 24 hours after the administration was calculated by the trapezoid method from the hypoglycemic rates at individual measurement points to determine the hypoglycemic rate area value (%·hr).

The compounds of Examples 1 to 7 had a hypoglycemic rate area value of 132 to 242 (%·hr) each. The specific acid addition salt of the compound (I) of the present invention has an excellent hypoglycemic effect and is useful as a medicine for treating or preventing hypertension or the like.

### (Preparation Example 1) Capsules

Powders of the example compound (10.0 mg), lactose (168.7 mg), corn starch (70.0 mg) and magnesium stearate (1.3 mg) (250 mg in total) are mixed and allowed to pass through a 60-mesh sieve. Then, the resulting powder was put in No. 2 gelatin capsules to prepare capsules.

### (Preparation Example 2) Tablets

Powders of the example compound (10.0 mg), lactose (149.0 mg), corn starch (40.0 mg) and magnesium stearate (1.0 mg) (200 mg in total) are mixed and tableted by a tableting machine to prepare tablets having a weight of 200 mg each.

INDUSTRIAL APPLICABILITY

The specific acid addition salt of 2-amino-1,4-dihydro-6-methyl-4-(3-nitrophenyl)-3,5-pyridinedicarboxylic acid 3-(1-diphenylmethylazetidin-3-yl) ester 5-isopropyl ester of the present invention has excellent calcium antagonistic effect, hypotensive effect, vasodilative effect, cardioprotective effect, antiarteriosclerotic effect, diuretic effect, nephropathy inhibitory effect and lipid peroxide generation inhibitory effect and is excellent as a pharmaceutical compound in terms of properties such as physicochemical properties, thermal stability, storage and handling stability, residual solvent ratio, hygroscopicity, deliquescence, solubility, pharmacological properties, pharmacokinetic properties, oral absorbability, concentration in blood, bioavailability, pharmacokinetics, safety and toxicity. Therefore, the acid addition salt is useful as a medicine, preferably a medicine for treating or preventing hypertension, heart disease, arteriosclerosis or nephropathy, more preferably a medicine for treating or preventing hypertension or heart disease, and most preferably a medicine for treating or preventing hypertension. Further, the specific acid addition salt of the compound (I) of the present invention may have excellent properties in that the concentration in blood varies only slightly according to a change in intragastric pH and is difficult to be affected by the diet. Therefore, the acid addition salt is useful as a medicine for a warm-blooded animal, and preferably as a medicine for a human.

BRIEF DESCRIPTION OF THE DRAWINGS

Figure 1 shows a powder X-ray diffraction pattern of 2-amino-1,4-dihydro-6-methyl-4-(3-nitrophenyl)-3,5-pyridinedicarboxylic acid 3-(1-diphenylmethylazetidin-3-yl) ester 5-isopropyl ester dihydrobromide obtained in Example 1;
Figure 2 shows a powder X-ray diffraction pattern of 2-amino-1,4-dihydro-6-methyl-4-(3-nitrophenyl)-3,5-pyridinedicarboxylic acid 3-(1-diphenylmethylazetidin-3-yl) ester 5-isopropyl ester dimethanesulfonate obtained in Example 3;
Figure 3 shows a powder X-ray diffraction pattern of 2-amino-1,4-dihydro-6-methyl-4-(3-nitrophenyl)-3,5-pyridinedicarboxylic acid 3-(1-diphenylmethylazetidin-3-yl) ester 5-isopropyl ester trimethanesulfonate obtained in Example 4;
Figure 4 shows a powder X-ray diffraction pattern of 2-amino-1,4-dihydro-6-methyl-4-(3-nitrophenyl)-3,5-pyridinedicarboxylic acid 3-(1-diphenylmethylazetidin-3-yl) ester 5-isopropyl ester di-p-toluenesulfonate obtained in Example 6;
Figure 5 shows a powder X-ray diffraction pattern of 2-amino-1,4-dihydro-6-methyl-4-(3-nitrophenyl)-3,5-pyridinedicarboxylic acid 3-(1-diphenylmethylazetidin-3-yl) ester 5-isopropyl ester monocitrate obtained in Example 2;
Figure 6 shows a powder X-ray diffraction pattern of 2-amino-1,4-dihydro-6-methyl-4-(3-nitrophenyl)-3,5-pyridinedicarboxylic acid 3-(1-diphenylmethylazetidin-3-yl) ester 5-isopropyl ester dibenzenesulfonate obtained in Example 5;
Figure 7 shows a powder X-ray diffraction pattern of 2-amino-1,4-dihydro-6-methyl-4-(3-nitrophenyl)-3,5-pyridinedicarboxylic acid 3-(1-diphenylmethylazetidin-3-yl) ester 5-isopropyl ester dinaphthalenesulfonate obtained in Example 7;
Figure 8 shows a powder X-ray diffraction pattern of 2-amino-1,4-dihydro-6-methyl-4-(3-nitrophenyl)-3,5-pyridinedicarboxylic acid 3-(1-diphenylmethylazetidin-3-yl) ester 5-isopropyl ester dihydrochloride obtained in Comparative Example 1;
Figure 9 shows a powder X-ray diffraction pattern of 2-amino-1,4-dihydro-6-methyl-4-(3-nitrophenyl)-3,5-pyridinedicarboxylic acid 3-(1-diphenylmethylazetidin-3-yl) ester 5-isopropyl ester dihydrochloride obtained in Comparative Example 2;
Figure 10 shows a powder X-ray diffraction pattern of 2-amino-1,4-dihydro-6-methyl-4-(3-nitrophenyl)-3,5-pyridinedicarboxylic acid 3-(1-diphenylmethylazetidin-3-yl) ester 5-isopropyl ester monosulfate obtained in Comparative Example 3; and
Figure 11 shows a powder X-ray diffraction pattern of 2-amino-1,4-dihydro-6-methyl-4-(3-nitrophenyl)-3,5-pyridinedicarboxylic acid 3-(1-diphenylmethylazetidin-3-yl) ester 5-isopropyl ester hemifumarate obtained in Comparative Example 4.

## Claims

1. A hydrobromide, citrate, methanesulfonate, benzenesulfonate, p-toluenesulfonate or naphthalenesulfonate of 2-amino-1,4-dihydro-6-methyl-4-(3-nitrophenyl)-3,5-pyridinedicarboxylic acid 3-(1-diphenylmethylazetidin-3-yl) ester 5-isopropyl ester.

2. The salt compound according to claim 1, which is a hydrobromide, methanesulfonate or p-toluenesulfonate of 2-amino-1,4-dihydro-6-methyl-4-(3-nitrophenyl)-3,5-pyridinedicarboxylic acid 3-(1-diphenylmethylazetidin-3-yl) ester 5-isopropyl ester.

3. The salt compound according to claim 2, which is a hydrobromide of 2-amino-1,4-dihydro-6-methyl-4-(3-nitrophenyl)-3,5-pyridinedicarboxylic acid 3-(1-diphenylmethylazetidin-3-yl) ester 5-isopropyl ester.

4. The salt compound according to claim 3, which is a dihydrobromide of 2-amino-1,4-dihydro-6-methyl-4-(3-nitrophenyl)-3,5-pyridinedicarboxylic acid 3-(1-diphenylmethylazetidin-3-yl) ester 5-isopropyl ester.

5. A crystal of the dihydrobromide of 2-amino-1,4-dihydro-6-methyl-4-(3-nitrophenyl)-3,5-pyridinedicarboxylic acid 3-(1-diphenylmethylazetidin-3-yl) ester 5-isopropyl ester according to claim 4.

6. The crystal according to claim 5, which shows main d spacing peaks at 16, 4.4, 3.9, 3.1 and 3.0 Å in a powder X-ray diffraction pattern obtained by irradiation with Cu Kα rays.

7. The salt compound according to claim 2, which is a methanesulfonate of 2-amino-1,4-dihydro-6-methyl-4-(3-nitrophenyl)-3,5-pyridinedicarboxylic acid 3-(1-diphenylmethylazetidin-3-yl) ester 5-isopropyl ester.

8. The salt compound according to claim 7, which is a dimethanesulfonate of 2-amino-1,4-dihydro-6-methyl-4-(3-nitrophenyl)-3,5-pyridinedicarboxylic acid 3-(1-diphenylmethylazetidin-3-yl) ester 5-isopropyl ester.

9. A crystal of the dimethanesulfonate of 2-amino-1,4-dihydro-6-methyl-4-(3-nitrophenyl)-3,5-pyridinedicarboxylic acid 3-(1-diphenylmethylazetidin-3-yl) ester 5-isopropyl ester according to claim 8.

10. The crystal according to claim 9, which shows main d spacing peaks at 12, 7.8, 6.1, 4.8 and 4.5 Å in a powder X-ray diffraction pattern obtained by irradiation with Cu Kα rays.

11. The salt compound according to claim 7, which is a trimethanesulfonate of 2-amino-1,4-dihydro-6-methyl-4-(3-nitrophenyl)-3,5-pyridinedicarboxylic acid 3-(1-diphenylmethylazetidin-3-yl) ester 5-isopropyl ester.

12. A crystal of the trimethanesulfonate of 2-amino-1,4-dihydro-6-methyl-4-(3-nitrophenyl)-3,5-pyridinedicarboxylic acid 3-(1-diphenylmethylazetidin-3-yl) ester 5-isopropyl ester according to claim 11.

13. The crystal according to claim 12, which shows main d spacing peaks at 11, 4.6, 4.4, 3.9 and 3.6 Å in a powder X-ray diffraction pattern obtained by irradiation with Cu Kα rays.

14. The salt compound according to claim 2, which is a p-toluenesulfonate of 2-amino-1,4-dihydro-6-methyl-4-(3-nitrophenyl)-3,5-pyridinedicarboxylic acid 3-(1-diphenylmethylazetidin-3-yl) ester 5-isopropyl ester.

15. The salt compound according to claim 14, which is a di-p-toluenesulfonate of 2-amino-1,4-dihydro-6-methyl-4-(3-nitrophenyl)-3,5-pyridinedicarboxylic acid 3-(1-diphenylmethylazetidin-3-yl) ester 5-isopropyl ester.

16. A crystal of the di-p-toluenesulfonate of 2-amino-1,4-dihydro-6-methyl-4-(3-nitrophenyl)-3,5-pyridinedicarboxylic acid 3-(1-diphenylmethylazetidin-3-yl) ester 5-isopropyl ester according to claim 15.

17. The crystal according to claim 16, which shows main d spacing peaks at 6.8, 4.9, 4.7 and 4.2 Å in a powder X-ray diffraction pattern obtained by irradiation with Cu Kα rays.

18. A pharmaceutical composition for treating or preventing hypertension, heart disease, arteriosclerosis or nephropathy, the pharmaceutical composition comprising the salt compound according to any one of claims 1 to 17 as an active ingredient.

19. The pharmaceutical composition according to claim 18 for treating or preventing hypertension.

20. Use of the salt compound according to any one of claims 1 to 17 for producing a pharmaceutical composition for treating or preventing hypertension, heart disease, arteriosclerosis or nephropathy.

21. The use according to claim 20 for producing a pharmaceutical composition for treating or preventing hypertension.

22. A method for treating or preventing hypertension, heart disease, arteriosclerosis or nephropathy, the method comprising administering a pharmacologically effective amount of the salt compound according to any one of claims 1 to 17 to a warm-blooded animal.

23. The method according to claim 22 for treating or preventing hypertension.

24. The method according to claim 22 or 23, wherein the warm-blooded animal is a human.
